# EUROPEAN PATENT APPLICATION

(11) **EP 1 512 332 A2**
(43) Date of publication of application: **09.03.2005**
(21) Application number: 04254896.6
(22) Date of filing: 13.08.2004
(51) Int. Cl.: A23L 3/04, A23L 3/00, A23L 3/02, A23L 2/46, A23C 3/023, A61L 2/00, A61L 2/04, B65B 55/06, B65B 55/02

(54) **Method and apparatus for continuous processing of packaged products**

(30) Priority: 13.08.2003 US 494754 P
(71) Applicant: Mars, Inc., McLean, VA 22101-3883 (US)
(72) Inventor: Cutler, Brent L., Vernon CA 90058-0853 (US); Dido, Jeannette, Hoboken NJ 07030 (US); Lin, Yah Hwa E., Cerrito CA 90703 (US); Rieger, Ron W., Cleveland TN 37312 (US); Knittweis, Eric, Sherman Oaks CA 91411 (US); Keen, Bruce, Chattanooga TN 37421 (US); Chisholm, Gary N., Rancho Palos Verdes CA 90275 (US); Collins, Thomas M., Nazareth PA 18064 (US); Jurgensen, Don, China Springs TX 76633 (US); LaFleur, Ted, South Pasadena CA 91030 (US)
(74) Representative: James, Anthony Christopher W.P.

(57) **Abstract**

The present invention is directed to a method for the pasteurization and/or sterilization of a continuous web of packages. The method includes the steps of providing a continuous web of individual packages; moving the continuous web through a first mechanical pressure seal into a pressure chamber; applying an increased pressure and temperature; and moving the continuous web through a second mechanical pressure seal out of the pressure chamber. The present invention is also directed to a continuous pressure seal apparatus and an apparatus for the pasteurization and/or sterilization of a continuous web of flexible packages. The apparatus includes a first mechanical continuous feeding pressure seal; a pressure chamber; and a second mechanical continuous feeding pressure seal. The first pressure seal is adjacent to an entry to the pressure chamber and the second pressure seal is adjacent to an exit of the pressure chamber.

## Description

### TECHNICAL FIELD

The present invention relates generally to processing a product within a package, and more particularly to a system and method of sterilizing unseparated sealed packages in continuous webs.

### BACKGROUND OF THE INVENTION

The efficiency of producing a packaged food product may be increased by providing a package in which the food product can be both processed and commercially distributed. Automated assembly lines for producing such packaged food products typically include a retort system in which the packaged food product is cooked and sterilized. In the conventional process for sterilizing packaged foods, packages are first filled with a food product and sealed. Thereafter, the sealed containers are heated in a pressure cooker or retort apparatus in batches to sterilize the packaged product.

Because these containers are sealed, it is required that the packages be heated under pressure so that the container does not burst. This is particularly true of flexible or semi-rigid packaging which requires a higher pressure to be exerted on the exterior of the package than that produced by the contents of the package as it is heated. This is called over pressure and is typically in the range of 10 - 50 p.s.i.g. Due to these high pressure requirements, cooking and sterilization processes for flexible and semi-rigid packages are generally carried out in batch retort apparatus which have been designed to be sealed shut and pressurized during heating. These packaged products are ordinarily placed in trays, cassettes, or bins which are then placed in the retort apparatus for a period of time.

A retort system not only cooks the product, but can be used to sterilize the product. Commercial sterilization is defined as the minimum temperature necessary to destroy Clostridium botulinum endospores while minimizing the alteration of the food product. A commercial sterilization process uses sufficient heat to reduce a population of C. botulinum by 12 logarithmic cycles. The required retort processing time for commercial sterilization depends upon a number of variables, such as the size of the food product and the temperature required to cook and sterilize the food product. Thus, the required retort processing time typically contributes significantly to the entire processing time.

The duration for the retort process is based on the length of time it would take to sterilize the coldest spot within a package as determined by a heat penetration test. Sterilization time is usually based on the time required to heat up to the proper sterilization value the center-most packages since these packages tend to reach the required temperature after the outer-most packages on the tray, cassette or bin reach the proper temperature. This is especially true where trays are stacked on one another, such that the inner packages take longer to reach the proper sterilization value as they would be in a typical batch retort system. Each package is adequately sterilized, but packages on the outer periphery of the tray are overheated in relation to the center-most packages which leads to overcooking or uneven cooking of the contents of the packages.

Batch processing of individual packages through the cooking/sterilization process is undesirable for a number of reasons. One of the reasons batch processing is undesirable is due to the mismatch in speed between the pouch filling apparatus and the cooking/sterilization process. A pouch filling apparatus can fill 600 packages per minute, while the cooking/sterilization step takes considerably longer. Thus, the individual packages are accumulated in trays, cassettes or bins prior to processing the packages through the cooking/sterilization step. A second reason batch processing through the cooking/sterilization process is undesirable is due to the fact that conventional retort systems include a large apparatus that requires a significant amount of time to heat and cool. Additionally, the volume of packaged products processed inside a conventional retort apparatus occupies less than 10% of the retort space because of the space occupied by the structural support of the trays, cassettes, bins and space between the individual packages.

In batch retorting, the mass of the products processed compared to the mass of both the carriers and the processing apparatus is also less than 10%. This means that at least 90% of the energy in heating and cooling is wasted in the batch operated retort processing system. There are also other problems associated with the retorting of individual packages that are batch loaded into a carrier. One problem is that the carriers, such as tray holders, pouch racks or cassettes, are designed to fit the product package dimension and shape. Thus, there is no interchangeability if different sized and dimensioned packages are used. Other problems include the time required to load and unload the carriers as each pouch must be handled individually, plus the labor or equipment required to load the cassettes/carriers into the retort apparatus.

A solution to the problem of long sterilization and uneven heating of packaged products, in particular canned products, has been addressed in U.S. Patent No. 5,301,603, to Mignogna et al. This patent describes a process and system for sterilizing packages of thermally-treatable products having different sterilization processing requirements. Generally disclosed is a chain-driven conveyance system for passing canned products through an optional pre-heat unit and then passing the products through a hydrostatic pressure cooker having towers for pre-heating, sterilizing and cooling.

The tower-based hydrostatic sterilizer, as disclosed in Mignogna and as conventionally used today for the sterilization and cooking of canned food products, requires significant capital costs not only in operation and maintenance, but the physical space necessary to operate such a sterilizer. The conventional hydrostatic sterilizer utilizes chain-driven trays or bins to move cans and packages through the sterilizer. Utilizing these trays requires that the towers or legs of the sterilizer be very wide to accommodate the trays. In order to achieve proper pressures, these towers require a certain height requirement such as for example up to 60 to 80 feet tall. The process disclosed in Mignogna and as conventionally used today for the cooking and sterilization of canned foods would require even greater heights for flexible or semi-rigid packages. This is because the pressure requirements are so high due to the overpressure that is required in the cooking/sterilization of flexible or semi-rigid packages in order to prevent the packages from bursting, which leads to even greater costs.

U.S. Application Serial No. 10/131,733, incorporated herein by reference, describes a method and apparatus for continuous thermal processing of individual packages. The application discloses a system for sterilizing individual packages in a hydrostatic pressure cooker utilizing a cable-driven conveyance mechanism. Though it solves the problem of loading and unloading found in batch systems, it utilizes hydrostatic pressure which causes it to suffer the same problem as the system described above.

Efficiency of the production process could also be increased by using an energy source that can cook and/or sterilize the packaged food product more quickly than can be accomplished with conduction heating as used in the typical retort process. One such known source is a microwave energy source, which readily could provide adequate energy in a fraction of the time. U.S. Pat. No. 3,335,253 is directed to an apparatus that provides for the microwave heating under hydrostatically derived pressure, thus eliminating the need for a mechanical pressure lock. This patent describes a typical hydrostatic retort apparatus that utilizes microwave energy instead of steam for the continuous cooking/sterilization processing of food products. In a hydrostatic retort apparatus, high towers or hydro-legs have to be constructed in order to provide sufficient pressure to overcome the internal vapor pressure buildup at high sterilization temperatures. As with any hydrostatic retort system, this apparatus requires significant capital costs not only in operation and maintenance, but the physical space necessary to operate such a sterilizer at the required pressures for flexible or semi-rigid packages.

U.S. Pat. Nos. 3,889,009 and 5,066,503 are directed to a method and U.S. Pat. Nos. 3,961,569 and 5,074,200 are directed to an apparatus for sterilizing food products in a continuous process using elecromagnetic or microwave energy. Individual containers of food or individual flexible packages are conveyed through a pressure apparatus that encloses and pressurizes a microwave heating section utilized to sterilize the individual containers or packages. The method of U. S. Pat. No. 5,066,503 is not a continuous process and as such it requires the labor of current batch sterilization systems because the individual packages must be loaded into the pallets and the pallets must be loaded into the pressure apparatus through an pressure seal mechanism into and out of the pressurization chamber.

In U. S. Pat. No. 3,961,569, the individual packages are conveyed through the pressure apparatus between a pair of thermally insulated conveyor belts that are permeable to the microwaves. The insulated conveyor belts are spaced apart from each other a distance about equal to the thickness of the packages and hold the heat within the individual packages as the packages move through the microwave heating section and the temperature maintenance section of the pressure apparatus. This system only allows for the passage of individual packages or packages through the rotary lock into and out of the pressure apparatus which results in relatively slow processing times. U.S. Pat. No. 5,750,966 discloses a plant for sterilizing solid or liquid packaged products using microwaves. The individual packages are conveyed through a number of tubular elements that are separately operable at different temperatures and pressures.

However, microwave cooking generally offers several challenges, such as the difficulties associated with ensuring evenly distributed and consistent cooking throughout the food product. Two patents that address this issue are U.S. Pat. No. 3,809,845 and U.S. Pat. No. 4,999,471. In U.S. Pat. No. 3,809,845 each product unit is enclosed in a casing that is transparent to electro-magnetic energy. The product unit is surrounded by a fluid medium that allows the entire product to reach a uniform desired temperature when heated in an electro-magnetic field. In U.S. Pat. No. 4,999,471 packaged food products are stabilized and the packaging is sanitized by heating the packages with microwave energy in a superatmospheric pressure. The food products travel through a microwave zone, a heated non-microwave zone and a cooling zone in which the air in all of the zones is thermostat regulated kept in motion by a fan.

Accordingly, it would be desirable to provide a system and method for the sterilization of continuous webs of flexible or semi-rigid packages that does not have the cost and time disadvantages associated conventional retort systems including batch or hydrostatic, which involve the use of trays, cassettes or bins. Such a system and method, with less structural support for the individual packages, could utilize any means of thermal energy for the sterilization.

It further would be desirable to provide a system and method that allows for the processing of a continuous web of packages in which the sterilization apparatus is always at the proper sterilization temperature and pressure and the continuous web of packages could continuously move into and out of the sterilization apparatus through a mechanical pressure lock system.

It would further be desirable to provide a system and method in which a continuous web of packages could move from a pouch filling station into a sterilization apparatus without requiring the additional steps or labor of placing the packages into carriers and then placing the carriers into the sterilization apparatus. Additionally, a single continuous web of packages would also be easier to control in a processing line than a multitude of individual packages.

### BRIEF SUMMARY OF THE INVENTION

The present is directed to a method for the pasteurization and/or sterilization of products. The method includes the steps of: a) providing a continuous web of individual packages containing the product; b) moving the continuous web through an entry continuous mechanical pressure seal into a chamber having an increased pressure and temperature; and c) moving the continuous web through an exit continuous mechanical pressure seal out of the pressure chamber. The entry and exit pressures seals provide for continuous feeding of the web.

The continuous web of packages is formed from material selected from the group consisting of flexible material, semi-rigid material and combinations of flexible and semi-rigid material.

The mechanism for moving the continuous web through the pressure seals and pressure chamber is selected from a group consisting of a clip and cable mechanism, dual carrier belts and a series of reels.

The method further includes the step of accumulating portions of the continuous web prior to moving the continuous web through the first pressure seal or within the pressure chamber. The method further includes moving the continuous web through a cooling region in the chamber. The method further includes the step of agitating the products in the continuous web while applying the increased pressure and temperature.

The entry and exit pressure seals are selected from a group consisting of a rotary belt valve, a continuous rotary door pressure seal and a split baffle system.

The continuous web of individual flexible packages is configured to include a reinforced area for preventing the deformation or tearing of the continuous web as it is pulled through a food processing line.

The present invention is also directed to an apparatus for the pasteurization and/or sterilization of product in a continuous web of packages that includes a) a chamber capable of containing an increase in pressure and temperature; b) the chamber having a continuous mechanical pressure seal for entry and exit into and out of the chamber; and c). a mechanism for continuous feeding of a web of packages into and out of the chamber.

The one pressure seal is configured as the entry and exit pressure seals and the chamber includes a mechanism for moving the web through the pressure seal and the chamber. The mechanism for moving the web through the pressure seal and chamber is selected from a group consisting of a clip and cable mechanism, dual carrier belts and a series of reels. The pressure seal is selected from a group consisting of a rotary belt valve, a continuous rotary door pressure seal and a split baffle system.

The invention is further directed to a pressure seal that includes a matched set of flexible belts; each belt being in the shape of a continuous tube; the belts being configured to rotate simultaneously whereby forming a rotating pressure seal.

In another embodiment of the invention, a pressure seal containment system includes at least one pressure containment loop; a plurality of baffle containment units, in which the units are connected to each other a predetermined spaced apart distance; and at least one pressure chamber. The plurality of baffle containment units are sized and shaped such that they form an air tight seal within the loop and provide for the continuous passage of a continuous web of packages into and out of the at least one pressure chamber.

The pressure seal containment system can include a first pressure containment loop and a first pressure chamber and a second pressure containment loop and a second pressure chamber connected to each other by a pressurized conduit. The first pressure chamber increases the pressure surrounding the continuous web and the second pressure chamber returns the web back to atmospheric pressure.

The containment units are formed from a compressable material capable of withstanding high temperatures and high pressures and can be cylindrical in shape and configured to open like clamshell.

Also provided is a system for multi-step food production processing comprising the steps of: a) providing a continuous web of individual packages containing said product; b) moving the continuous web through an entry continuous mechanical pressure seal into a chamber having an increased pressure and temperature; and c) moving the continuous web through an exit continuous mechanical pressure seal out of the pressure chamber.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

FIG. 1 is a block diagram of an embodiment of the various steps for processing a food product;

FIG. 2 is a perspective illustration of one embodiment of a pouch forming, filling, sterilizing and packaging process;

FIG. 3 is a block diagram of the pasteurization/sterilization step of the present invention;

FIG. 4 is perspective illustration of a series of accumulation reels containing portions of the continuous web of the present invention;

FIG. 5 is a cross sectional view of one embodiment of an accumulation mechanism of the present invention;

FIG. 6 is a perspective illustration of a rotary belt valve of the present invention;

FIG. 7 is a perspective view of a continuous rotary door pressure seal of the present invention;

FIG. 8 is a cross sectional view of an alternative embodiment of the rotary door pressure seal of the present invention;

FIG. 9 is a cross sectional view of one embodiment of a pressure chamber of the present invention;

FIG. 10 is a perspective illustration of a dual carrier belt conveying mechanism of the present invention;

FIG. 11 is a cross sectional view of another embodiment of pressure chamber of the present invention illustrating an alternate conveying and heat transfer mechanism;

FIG. 12 is a cross sectional view of another embodiment of pressure chamber of the present invention illustrating an alternate conveying and heat transfer mechanism;

FIG. 13 is a cross sectional view of an embodiment of the pressure chamber of the present invention illustrating the utilization of one pressure seal mechanism; and

FIG. 14 is a perspective view, in partial cross section, of another embodiment of the pressure seals and pressure chamber of the present invention illustrating a split baffle system;

FIG. 15 is a perspective view, in partial cross section, of a portion of the pressure seal of Fig. 14;

FIG. 16 is a perspective view, in partial cross section, of a portion of the pressure seal of Fig. 15; and

FIG. 17 is a perspective view of an open baffle and the continuous web of the split baffle system of Fig. 14.

### DETAILED DESCRIPTION OF THE INVENTION

The present invention is directed to a system and method for the pasteurization and/or sterilization of continuous webs of packaged products. The packaged products can include any flexible or semi-ridged packaging or a combination of flexible and semi-ridged packaging in which the individual packages are connected in a continuous web. One skilled in the art will know that connected means that the individual packages can have a solid connection, a perforated connection or a small attachment connection. The continuous web can also be formed by connecting individual packages to a clip and cable mechanism or by attaching the individual packages to a continuous rope. Preferably, at least a portion of the packaging is transparent to microwave energy. Examples of the packages can include flexible pouches, sectioned trays covered with a heat sealed transparent material, bowls, such as rice bowls, covered with a heat sealed material, and cups, such as yogurt cups with an appropriate cover. While the description is principally directed to human and pet food products, the method and system can be equally useful for the production of pharmaceutical products, medical devices and agricultural products.

Such a system and method could utilize any thermal energy source, such as steam, hot air, conduction heating and thermal radiation for pasteurizing and/or sterilizing the packaged products under pressure. The system could also utilize any thermal energy source in combination with other pasteurization and/or sterilization process. A preferred energy source is microwave heating, however any combination of thermal energy sources could be used such as steam and microwave heating or steam and hot air. If a microwave energy source is utilized for the thermal energy source, one skilled in the art would be aware of the parameters surrounding microwave heating. For example, microwave energy often can result in uneven heating on the surface and throughout the product. Further, the product must have sufficient moisture content such that it is amenable to heating upon application of microwave energy. Susceptor lined food packages allow the food in the package to reach higher temperatures (e.g., 350-400° F), whereas food in conventional package materials typically reach lower temperatures (e.g. 200-275° F). In many manufacturing processes, the food product is packaged in a microwave appropriate container or package, which is one that does not melt, spark or deform during microwave use. A microwave appropriate container also retains its structure during microwave heating or cooking.

The present invention is distinguishable from known methods of both conventional retort and microwave sterilization systems in that it utilizes a continuous web of packages that are continuously processed through a pressurize chamber via a continuous mechanical pressure seal rather than processing individual packages through a pressurized microwave heating chamber or a web of packages through a hydrostatic sterilization apparatus. The continuous mechanical pressure seal is a pressure seal that provides for the continuous movement of a web of packages through a pressure seal in which the pressure seal mechanism is based on mechanics as opposed to a pressure seal based on a hydrostatic system. The continuous webs of packages can includes as few as two individual connected packages or substantial volumes of connected packaged products, connected by means of a solid connection, a perforated connection, a small attachment connection, a clip and cable mechanism or continuous rope. Continuous sterilization uses about one forth the energy of batch sterilization for two reasons. First, the energy goes into pasteurizing/sterilizing the product rather heating up the trays or cassettes, the conveyors, and the sterilization apparatus. Second, the system continuously remains at pasteurization/sterilization conditions as opposed to heating up and cooling down with every cycle, thus reducing the overall pasteurization/sterilization time. Additionally, the packages in a continuous system are processed in direct contact with the thermal cooking/sterilization process, rather than surrounded by the stainless steel cassettes or trays.

The inventive method and system can be used to produce and process any fluid, gelled, dry, moist, and semi-moist human and pet food products, including snacks and treats, and pharmaceutical products, medical devices or agricultural products, all packaged in hermetically sealed continuous webs of packages. The human food products can include meat and vegetable chunks in a sauce, meat chunks or vegetable pieces with or without a sauce, rice or pasta products with or without a sauce or any combination thereof. However, the process described below is generally directed to a semi-moist chunk type food packaged with a gravy or sauce in a sealed pouch container. The processes for forming dry and moist food products, snack food and pet food are well known to those skilled in the art of manufacturing edible products.

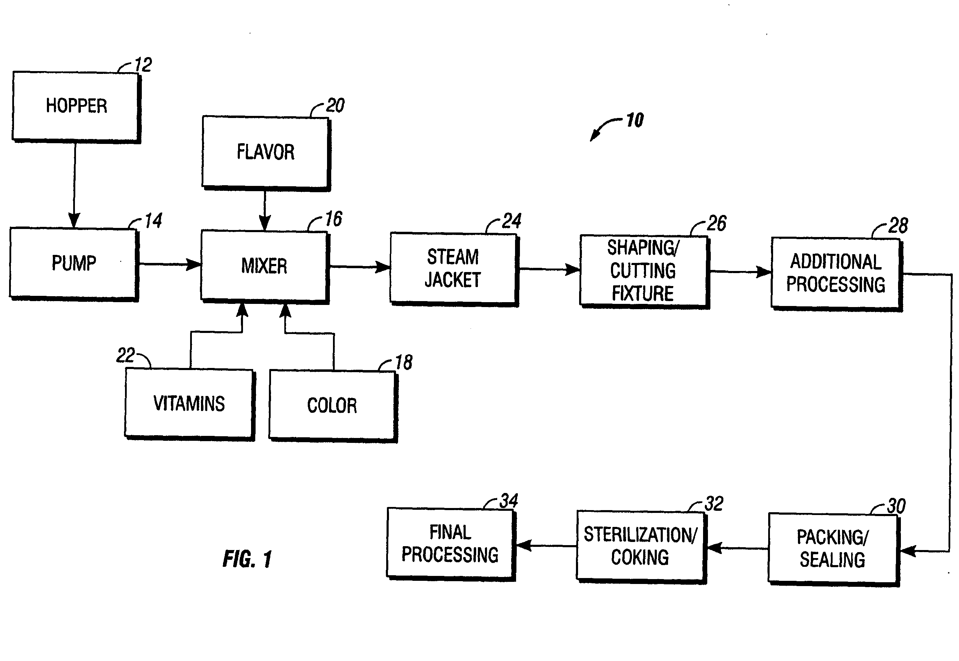

A block diagram as shown in Fig. 1, represents one embodiment of the various steps for processing a food product in a package capable of being processed in a wet or dry high temperature, high pressure environment. The steps will be described with reference to an automated assembly line in a food processing facility. However, it should be understood that an automated assembly line is not necessary to practice the invention described herein, and that all or a subset of the steps may be performed in a non-automated manner. It should be further understood that the invention is applicable to the processing of other products such as pharmaceutical products, medical devices or agricultural products that could also benefit from the principles described herein.

Fig. 1 illustrates one embodiment of an exemplary processing system 10 for producing a packaged, sterilized product (e.g., a food product). In the example discussed, the food product may be a meat product, dairy product, starch-based product (e.g., rice, dough, pasta), etc. Generally, processing of the food products begins with placing one or more ingredients (e.g., meat) into a hopper 12. A pump 14 pumps the contents of the hopper 12 into a mixer 16 where a variety of other ingredients may also be introduced. For example, it may be desirable to mix the starting ingredient with one or more of a coloring agent 18, flavoring agents 20, and one or more vitamins 22. The ingredients are combined in the mixer 16 for a time period sufficient to adequately distribute all ingredients throughout the resultant mixture.

The resultant mixture is then transported from the mixer 16 to the next appropriate processing station. For example, the mixture may be transported through a steam jacket 24 which may inject steam into the mixture to introduce moisture. Further, if the mixture is a meat or flour-based product, the mixture may be transported to a shaping/cutting fixture 26 configured to impart a particular shape and size to the product. After exiting the fixture 26, the shaped/cut product then may be subjected to further processing 28 (e.g., addition of nutrients, coloring agents, etc.) before packaging by an appropriate packaging and sealing device 30.

Alternatively, the meat chunks or pieces can be provided ready-made for dispensing into the packages, such as diced or cubed chunks of beef, chicken, lamb, veal, pork or fish. Meat chunks include any meat, poultry, fish product or combination thereof. Thus, in this embodiment of the method and system, a meat preparation module (not shown) would include the placement of a variety of ready-made meat pieces and/or vegetables and/or rice and pasta products into an appropriate hopper for each different product as would be known to one skilled in the art of food processing. Any additional processing of these products would take place in the hopper or a mixing apparatus.

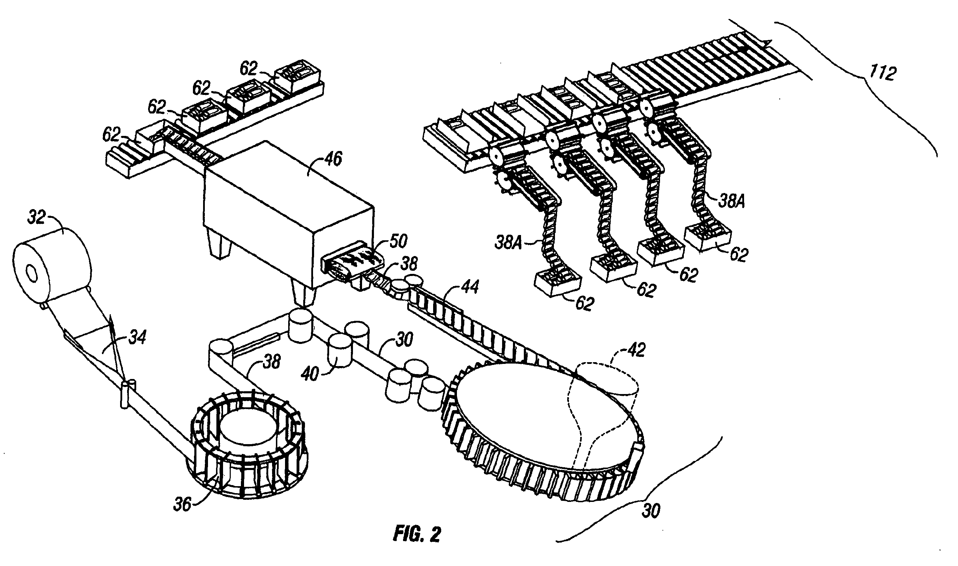

The package forming module includes a separate, but integral, processing line configured for the formation of the packages from a heat sealable, continuous plastic film (Fig. 2). A description of the preferred film or pouch material is discussed below. In a typical pouch forming process known to one skilled in the art, rolls of film 32 are provided in which the film is fed through a plow 34 in order to fold the sheet of film. The vertical sides of the folded film 36 are sealed as well as the bottom of the pouch if necessary. This process creates a continuous web of individual packages 38. A portion of the continuous plastic film can be printed with text and graphics that would be appropriate for any variety of food product. In another embodiment, the processing line can utilize non-printed film in which the processing line would include a programmable high speed printing system 40 for imprinting the text and graphics on the front and back of each of the packages in the continuous strip as it runs through the printing system portion of the pouch forming line.

Suitable material for forming the packages can include polyethylene terephthalate film or sheets, polypropylene film or sheets, foamed polypropylene, and foamed polyethylene terephthalate. Semi-ridged packaging material can include for example, paperboard, corrugated board (micro-flute, E, F, C or B shaped flute or any other fluted board), paperboard canister, plastic sheeting such as polyethylene terephthalate (PET). The paperboard could be laminated with a number of films such as susceptor film, PET, polypropylene. These materials can also be coated or laminated in order to prevent moisture absorption. Any form of polyester would also be suitable as a semi-rigid or flexible material. The material for forming the continuous strip of packages also may include an oxygen or moisture-barrier material or layer, such as a thermoplastic synthetic resin, for example polyvinylidene chloride (PVDC) or ethylene-vinyl alcohol copolymer (EVOH). *See* U.S. Pat. 4,435,344, which is incorporated by reference.

The food production line 10, at the completion of the meat preparation and forming module 26 or the completion of the meat preparation module, converges with the pouch forming line at the filling and sealing module 30. At the filling and sealing module 30, an electronically controlled filling system 42, as is well known to one skilled in the art, deposits a measured amount of food product into each of the packages 38B in the continuous web of packages 38. More than one filling station can be utilized. Thereafter, the tops of the packages are sealed 44. The continuous web 38 of packages is then transported to a pressure chamber 46. After the continuous web 38 of packages leaves the pressure chamber 46, the web can be accumulated in work boxes 62 and then transported to a final packaging module 112 for packaging for commercial sale.

In a preferred embodiment, either the food product can be pre-heated prior to being deposited in the packages or the filled packages can be pre-heated prior to entering the pressure chamber. The step of pre-heating can be accomplished by any thermal process such as direct steam injection or jacketed heating. This pre-heating step results in the reduction of the time required for sterilization to occur.

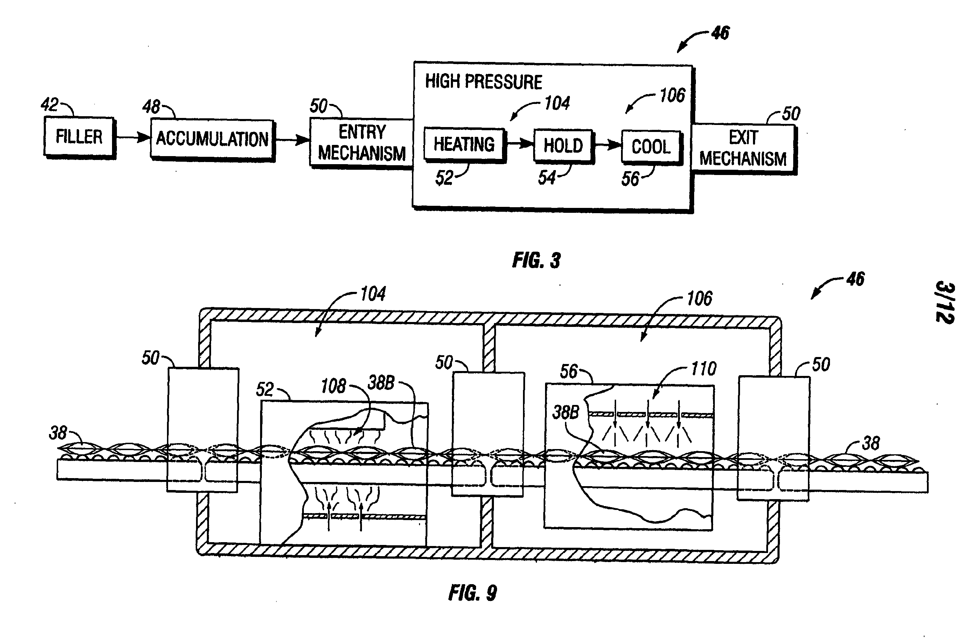

A block diagram as shown in Fig. 3, represents one preferred embodiment of the pasteurization/sterilization process of the present invention. As illustrated in Fig. 3, after the continuous web 38 leaves the filling and sealing module 30 of the food production line 10, the continuous web 38 enters an accumulation step that includes an accumulation mechanism 48 for handling the mismatch in speed between the pouch filling apparatus and the pasteurization/sterilization step. From the accumulation step the continuous web 38 is run through a continuous mechanical pressure seal 50 into a pressurized chamber 46 in which the pasteurization/sterilization step occurs. The pressurized chamber 46 includes a heating mechanism 52 for heating the packaged products, a holding step/mechanism 54 for holding the packaged products at a desired temperature and a cooling step/mechanism 56 for cooling the continuous web 38 of packaged products. In an alternate embodiment, the web 38 can enter an accumulation step 48 after heating in which the accumulated sections of the continuous web 38 would then enter the cooling step/mechanism 56 as a single continuous web 38. Thereafter, the continuous web 38 exits the pressure chamber 46 through a continuous mechanical pressure seal 50. The continuous mechanical pressure seal 50 can be configured to function as both an entry and exit pressure seal or the entry and exit continuous mechanical pressure seal can be provided by two pressure seal mechanisms 50. The pressure chamber 46 can also include a transitional continuous mechanical pressure seal 50.

The current practice for entering and exiting vessels of differential atmospheric pressure is the use of slide gate valves, rotary air lock valves or a combination of gates/doors forming a discontinous process. The alternative widely used in thermal processing is to go through a hydrostatic lock dependent on a column of water. The product is carried through the column of water which maintains the differential pressure in the pressure chamber by the differences in weights/heights of the two sides of the column of water.

As discussed above, one of the difficulties associated with the processing of a continuous web of individual packages is the mismatch in speed between the pouch filling apparatus and the pasteurization/sterilization step. A pouch filling apparatus can fill 600 packages per minute, while the pasteurization/sterilization step can take up to two minutes per pouch. The accumulation step/mechanism 48 provides for the accumulation of portions of the continuous web 38 in a holding section while keeping the packages in a single strand for processing through the pressure chamber 46. The accumulation mechanism 48 can be any device or apparatus that is designed to allow for the accumulation of portions of the continuous web 38.

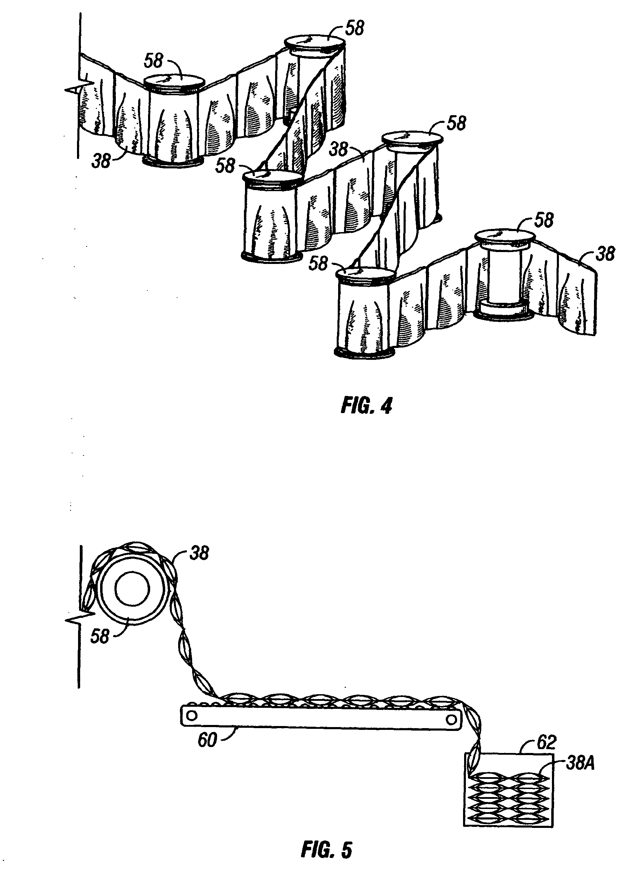

In one embodiment, the accumulation mechanism 48 can be configured as at least two reels 58 and can include any increasing number of reels 58 configured to accommodate the width and length of portions of the continuous web 38 (Fig. 4). The reels 58 would accumulate sections of the web 38A prior to the continuous web 38 entering the pressure chamber 46 in which the reel closest to the pressure chamber would feed the continuous web into the pressure chamber. The reels 58 could also be used to accumulate sections of the web 38A after the heating step and prior to entering the cooling step.

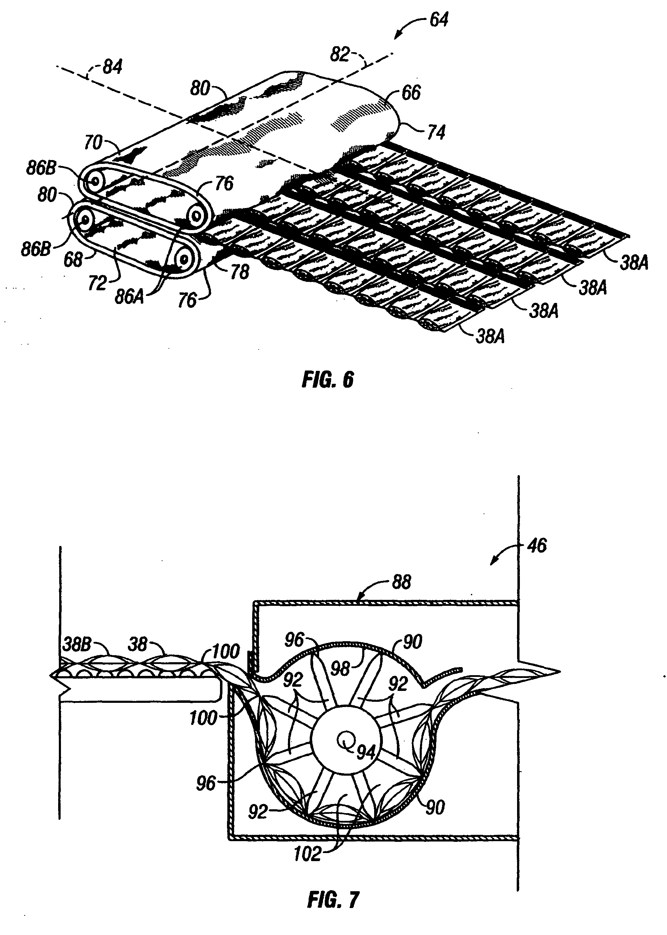

Alternatively, sections 38A of the continuous web 38 could be accumulated in a variety of apparatus including single or a plurality of gaylords, work boxes, accumulation conveyors, store-veyors and other methods known to those skilled in the art as long as the packages are able to be easily pulled out of the apparatus in a single strand. For example, as the continuous web 38 comes off a reel 58 it can feed on to a conveyor belt 60 that would deposit the sections 38A of continuous web into work boxes 62 (Fig. 5). The work boxes 62 containing sections 38A of the continuous web would then be conveyed to the pressure chamber 46 and the web 38 would be fed through the continuous mechanical pressure seal 50 into the pressure chamber 46. Additionally, a number of reels 58 could be used to simultaneously feed a plurality of web sections 38A through the pressure chamber 46. For example, four sections 38A of continuous webs 38 could be simultaneously processed through the pressure seal mechanism 50 and into pressure chamber 46 (Fig. 6). In another embodiment, festooning (not shown) could be used to accumulate and/or hold the continuous web of packages. Festooning devices are well know to those in the art and generally consist of a series of small trolleys called cable trolleys that are linked together by a cable or air hose. The cable trolleys travel along a wire rope or a channel that runs parallel to a bean. The continuous web of packages are looped on saddles on the cable trolleys which allow continuous lengths of webs to be accumulated and/or held in relatively short horizontal distances.

The continuous mechanical pressure seal 50 of the present invention provides for the continuous passage of one or more the webs 38 into and out of the pressure chamber 46. The inventive continuous mechanical pressure seal 50 can include several different configurations and can be altered to accommodate any shape or form of packaging. In one embodiment, a rotary belt valve 64 is utilized as the entry and exit continuous mechanical pressure seal 50 (Fig. 6). The rotary belt valve 64 can be formed from an flexible belting material. In one embodiment, the rotary belt valve 64 can be formed from flexible closed cell or visco-elastic cell materials. Alternatively, the rotary belt valve 64 can be formed from a matched set of flexible bladders formed from a flexible rubber-like material and filled with a malleable material such as a liquid or pressurized with gas/air. The rotary belt valve 64 includes a matched set of flexible belts 66, 68 that create a pressure seal by closely conforming around the individual packages of the continuous web 38 as it passes through the belts 66, 68. The belts 66, 68 are similar to large tractor tire inner tubes and preferably have an elongated rectangular shape. Each belt 66, 68 has an outside and inside surface 70, 72, a first and second end 74, 76, and a first and second side 78, 80. The belts 66, 68 also have an X axis 82 parallel with the first and second ends 74, 76 and a Y axis 84 parallel to the first and second sides 78, 80.

A spaced apart set of rollers 86A, 86B is positioned on the inside surface 72 of each of the belts 66, 68 in parallel alignment with the X axis 82. One set of rollers 86A is parallel and adjacent to the first side 78 and a second set of rollers 86B is parallel and adjacent to the second side 80 of each belt 66, 68, creating a tension between the first and second sides 78, 80 of the belts 66, 68. The set of rollers 86A, B are configured to allow each belt 66, 68 to rotate around an axis parallel to the X axis 82. The two belts 66, 68 are stacked one above the other forming a pinchless pressure seal area that is able to conform to any shape, format or pitch of products as required. The two belts 66, 68 rotate into each other such that the continuous web 38 is pulled in between the first sides 78 of the belts 66, 68, through the rotary belt valve 64 and out between the second sides 80 of the belts 66, 68 and into the pressure chamber 46. The continuous web 38 is pulled through the rotary belt valve 64 parallel to the Y axis 84 and perpendicular to the X axis 82. The mechanical rotary belt valve is a self feeding valve and mechanism. The rotary belts 66, 68 are capable of a very high turn down ratio and can run at a few feet per minute or up to the limits of a rotational speed that would be required for a continuous food production line. The rollers 86A, B of the belts 66, 68 can be driven by any rotary drive mechanism known to one skilled in the art of rotary mechanics.

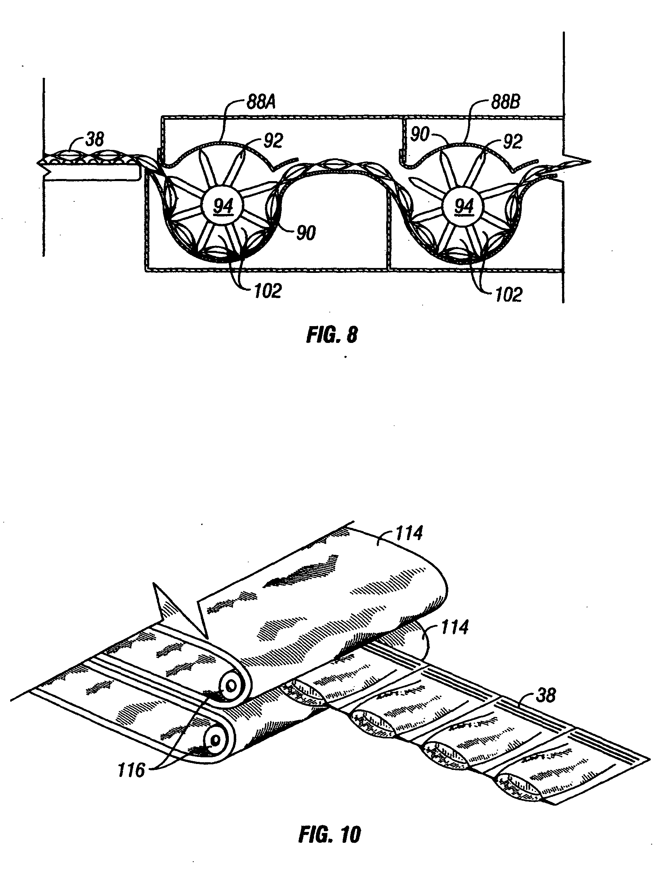

Another embodiment of the continuous mechanical pressure seal 50 is a rotary door pressure seal 88 that is configured similarly to a revolving door, having two opposed nearly semi-circular walls 90 within which a number of door elements 92 attached to a central axle 94 rotate. The outer edges 96 of the door elements 92 fit snugly against the inner surfaces 98 of the nearly semi-circular walls 90 (Fig. 7 and 8). The pitch of the door elements 92 matches the pitch of the individual packages 38B in the continuous web 38, such that the outer edge 96 of each door 92 feeds the web 38 at the seal 100 between each individual package 38B or groups of individual packages in the web 38. The rotation of the door elements 92 permits the continuous feeding of the web 38 into the compartments 102 between each of the door elements 92 from which point, rotation of the door elements 92 of the rotary door pressure seal 88 carries the continuous web 38 into the pressure chamber 46 while maintaining an effective seal for the pressure chamber 46. The rotary door pressure seal 88 of Fig. 7 is preferably used when the pressure change between the atmosphere and the pressure chamber 46 is less than or equal to 30 p.s.i.g. If the pressure change is greater, it may be preferable to use two rotary door pressure seal mechanisms in series 88A, 88B as illustrated in Fig. 8. The first rotary door pressure seal 88A could hold a pressure from 0 to 25 p.s.i.g. and the second pressure seal 88 B could hold a pressure from 25 p.s.i.g. to 50 p.s.i.g.

Upon entering the pressure chamber 46, the continuous web 38 of packaged products is subjected to an air pressure sufficient to retain the packaging material in substantial contact with the packaged contents. At least some pressure should be maintained for both the heating and cooling steps. This is necessary in order to balance the pressure buildup in the package, as pressure buildup will cause the package to burst open. In an exemplary embodiment, the air pressure is from about 10 to 50 p.s.i.g., preferably from 15 to 40 p.s.i.g., and more preferably from 20 to 35 p.s.i.g., which should be an amount sufficient to at least substantially counterbalance the internal pressure in the package resulting from vapor expansion during the thermal process. Application of the external pressure during heating preserves the shape and structure of the packaged contents, because expansion of the contents is restrained. This external pressure also assists in more efficient and even transfer of thermal energy to the contents of the package by keeping the packaging in contact with its contents.

In one embodiment, the pressure chamber 46 includes a first and second region 104, 106 and three continuous mechanical pressure seals 50 (Fig. 9). Upon entering the first region 104 of the pressure chamber 46, the continuous web 38 of packages is subjected to a thermal energy source 108. Preferably, the thermal energy source 108 provides for a temperature range of about 110 to 140° C. The individual packages 38B of the continuous web 38 remain in the first region 104 for a time sufficient to ensure that the package contents are thoroughly pasteurized and/or sterilized. The duration the packaged products are kept in the first region 104 can include both a time for increasing the temperature of the food product and a time for holding the food product at the desired temperature necessary for pasteurization and/or sterilization. In another embodiments, the pressure chamber 46 can include more than two regions. For example, one region may be provided for pre-heating the continuous web 38 of packages, a second region is provided for the heating step, a third region may be provided for the holding step, and a fourth region is provided for the cooling step. The cooling step may also take place in more than one region.

As would be readily recognized by one of skill in the applicable art, the time needed to ensure pasteurization and/or sterilization will vary depending on many different factors, including the amount of thermal energy applied, the volume of the first region, the type of product, the dimensions (e.g., thickness) of the package contents, etc.

In this first region 104, it is preferred that the webs 38 are held in a manner such that substantially all of each package 38B is exposed to the heat transfer medium 108. Since the packages are not overlapping or stacked during the heating step, the heating time would be decreased as it will take less time for the packages to reach the target temperature. After the heating step and during the hold time required to reach proper pasteurization or sterilization, the webs 38 can be held in an accumulation apparatus as describe above until being pulled as a singular strand directly into the second region 106 for cooling or pass through a transitional continuous mechanical pressure seal 50 into the second region 106 for cooling. Again the web 38 would be exposed to the cooling medium without overlap or stacking of the packages in order to provide for the quickest cooling time.

Once the portion of the continuous web 38 of packages being conveyed through the pressure chamber 46 has received adequate thermal exposure to ensure proper pasteurization and/or sterilization, the portion of web 38 is moved into the second region 106 of the pressure chamber 46 for a cooling-off period because the package contents retain significant heat after thermal exposure. The length of the cooling-off period will be such that the contents have cooled sufficiently such that upon removal of the external pressure, the individual packages will not rupture due to increased internal pressure from vapor expansion. In the embodiment illustrated in Fig. 9, the continuous web 38 of packages is conveyed through the first region 104 of the pressure chamber 46 through a transitional mechanical pressure seal 50 to a second pressurized cooling region 106 of the pressure chamber 46. It is not necessary that the pressure chamber 46 include a transitional mechanical pressure seal 50 as the different regions in the pressure chamber can be separated by other means or be configured such that no separation of the regions is necessary.

The pressure within the second region 106 can be either the same pressure as the pressure within the first region 104, or it may be greater or less than the pressure in the first region. In any event, an externally applied pressure is applied to the packages until the temperature of the package contents has cooled sufficiently to ensure that the individual packages will not rupture when returned to atmospheric pressure.

Any cooling mechanism can be utilized to lower the temperature of the contents of the packages, such as spray cooler, utilizing water or liquid nitrogen/carbon dioxide. If water is utilized as cooling mechanism, the water can be recycled to a pre-heat region in which the heat in the water can be utilized pre-heat the packages prior to the heating step. The system and method of the present invention is designed such that the packages are cooled down more rapidly than in other systems. This shortened cool down provides a number of advantages. One advantage is that it prevents degradation of the package contents, which is important for pharmaceutical products that include bio-active components. A second advantage is that the shortened cool down time reduces total processing time for the continuous web of packages.

In the embodiment illustrated in Fig. 9, the continuous web 38 of packages is returned to atmospheric pressure by conveying the continuous web 38 of packages from the pressure chamber 46 through an exit continuous mechanical pressure seal 50. Upon exiting the chamber 46, the continuous web 38 of packages may be subjected to any desired final processing steps, for example the final packaging module 111 as illustrated in Fig. 2. This module can include drying, separating the packages from the continuous web, application of an overwrap made of an oxygen and/or moisture-barrier material, placement of the sterilized packages in appropriate boxes or containers, etc. as suitable for commercial distribution.

As illustrated in the block diagram of Fig. 3, the pressure chamber 46 can include one pressurized chamber with two regions 104, 104 and two pressure seal mechanisms 50. In this embodiment, the webs 38 are held in a heating region 104 such that substantially all of each package is exposed to the energy source for the time required to reach proper pasteurization or sterilization. After the portion of the continuous web 38 of packages being conveyed through the pressure chamber 38 has received adequate thermal exposure, the portion of web is moved into a second region 106 of the pressure chamber 46 for a cooling-off period. Upon exiting the chamber 46, the continuous web 38 of packages may be subjected to any desired final processing steps such as described above.

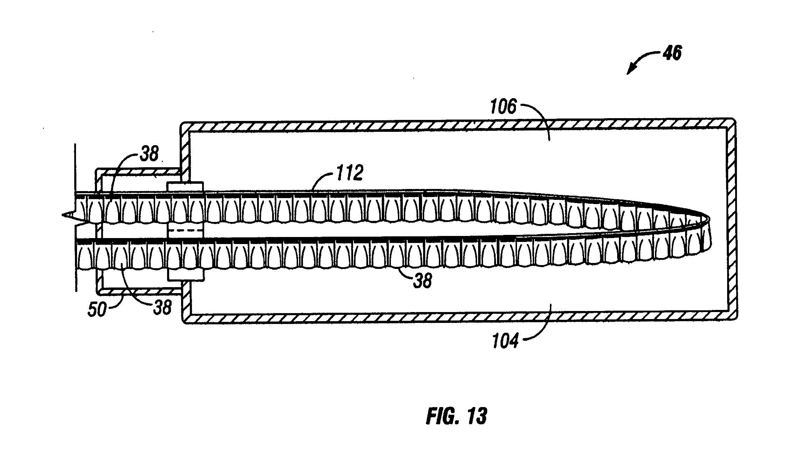

Fig. 13 illustrates a pressure chamber 46 utilizing only one pressure seal mechanism 50. In this embodiment, the continuous web 38 of packages could be attached to a cable mechanism 112 that enters and exits the pressure chamber 46 through one pressure seal mechanism 50. A portion of the continuous web 38 is held in a heating region 104 such that substantially all of each package is exposed to the energy source for the time required to reach proper pasteurization or sterilization. After the portion of the continuous web of packages being conveyed through the pressure chamber 46 has received adequate thermal exposure, the portion of web 38 is moved into a second region 106 of the pressure chamber 46 for a cooling-off period. Upon exiting the chamber 46, the continuous web 38 of packages may be subjected to any desired final processing steps such as described above.

In the present invention, it is desirable to have a mechanism that is configured to effectively pull the continuous web of packages through the entire food processing line without deformation or tearing of the packaging material. In one embodiment, a clip and cable mechanism, as described in U.S. Application Serial No. 10/131,733, could be used. The '733 application describes a unique package clip for attaching the continuous web of packages to a cable or a rail that includes a rail assembly and a clip assembly. The rail assembly is preferably shaped rectilinearly or with track grooves such that the rail assembly is moveable on a rail or a track. The rail assembly has a rail head with a slideable attachment arm positioned in the rail head. The slideable attachment arm is spring driven such that spring force maintains the attachment arm in a closed positioned. The package clips are attached to a cable by moving the attachment arm into an open position and placing a hook portion of the attachment arm around the cable. Tension from the spring will cause the hook to engage the cable. After the continuous webs of packages are attached to the package clips and the package clips are attached to the cable, the continuous webs of packages are simply conveyed through the pressure chamber. Individual packages attached to the clip and cable mechanism can form the continuous web of packages as contemplated in the present invention.

In order to prevent deformation or tearing of the package material as it is pulled through the entire food processing line, a high temperature, high tensile strength plastic rope can be welded to the surface of the top seal area of the packages in the web. The plastic rope would be melted into the surface of the top seal area of the packages. The plastic rope can be utilized to attach individual packages together to form the continuous web of packages as contemplated in the present invention. This rope could optionally be cut and molded at the packaging module in order to provide a handle for a segment of the strip of packages and to provide a easy tear start point for removing an individual pouch from the segment and for opening an individual package. Alternatively, the edges of the film used to form the packages could be reinforced by a continuous plastic band along the top of the continuous web. Regardless of the pulling mechanism used, it is configured to provide a constant feed for the web which would keep the web continuously threaded on the conveying mechanism.

In another embodiment, dual carrier belts 114 could be utilized as the conveying mechanism for the continuous web 38. The dual carrier belts 114 would include spring loaded tension devices 116 that provide exact positioning of the continuous web 38 and a self feeding mechanism that would eliminate the need for threading the web on a track mechanism (Fig. 10). The dual carrier belts 114 can be formed from any flexible belting material. In one embodiment, the belts 114 cane be formed from a flexible rubber-like material and filled with either a liquid or pressurized with gas/air. Alternatively, the carries belts 114 can be formed from flexible closed cell or visco-elastic cell materials.

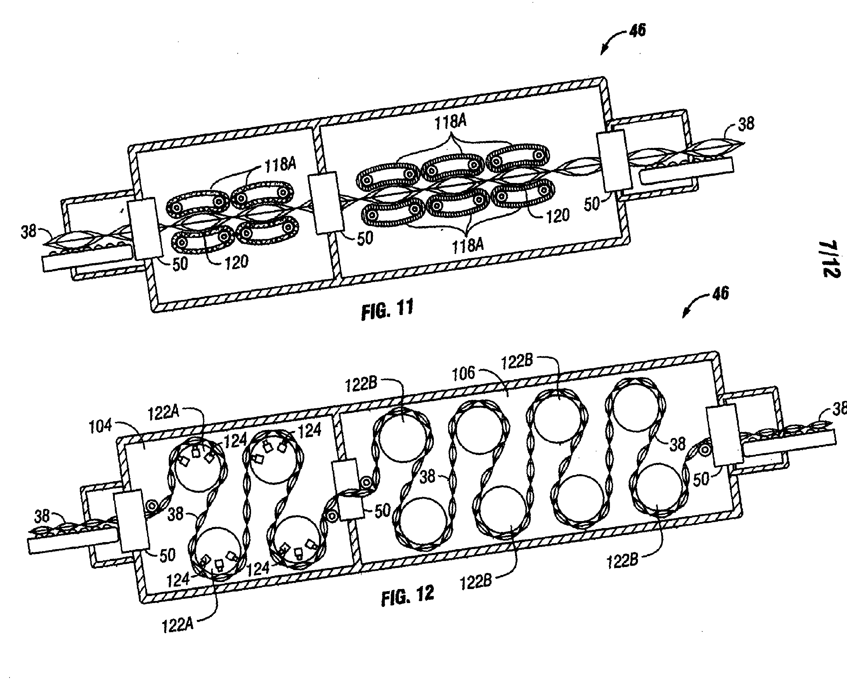

If the carrier belts 114 are formed from flexible closed cell or visco-elastic cell materials, a series of the dual carries belts 118 could also be utilized as the pressure control and the heat transfer mechanism for the continuous web 38 (Fig. 11). The series of dual belts 118 are configured to form a three-dimensional cavity 120 around the continuous web. The closed cells, preferably formed from viscoelastic polymer foams, could be filled with a gel having a high heat transfer coefficient and a boiling point above a product target sterilization temperature for heating and a freezing temperature below a product target cooling temperature to maintain flexibility during cooling and bending around conveyor pulleys. The continuous web 38 would pass through a continuous mechanical pressure seal 50 into a first set of dual carrier belts 118A in the pressure chamber 46 in which the contents of the packages would be heated to the appropriate sterilization value and the web 38 would then pass through a second series of dual carrier belts 118B that cool the packages to the appropriate cool down temperature. Upon exiting the pressure chamber 46 , the continuous web 38 would be subject to any desired final processing steps as described above. In this embodiment, the pressure chamber could utilize one, two or three pressure seal mechanisms as the entry, exit and transitional pressure seals 50.

An alternate embodiment for moving the continuous web through the pressure chamber includes a series of parallel reels 122 through which the continuous web 38 would wind (Fig. 12). The series of reels 122 would allow larger sections of the continuous web 38 more time to reside in the pressure chamber 46 without slowing down the pasteurization/sterilization process. Additionally, the up and down movement of the continuous web 38 as it moves through the various reels 122 results in agitation of the product in the individual packages 38B in the continuous web 38. The agitation facilitates heat transfer between cold spots and hot spots in the product. This allows for more uniform heating, a shortening of the heating cycle and a reduction in the temperature gradient. A first series of reels 122A in the first region 104 of the pressure chamber 46, includes microwave magnetron elements 124 configured to provide the required thermal exposure to ensure proper pasteurization and/or sterilization. If non-microwave thermal energy is used, the thermal source can include resistance heating, such as ohmic heating, or conduction heating through a heat transfer device.

In the exemplary embodiment illustrated in Fig. 12, the continuous web 38 of packages is conveyed through the first region 104 of the pressure chamber 46 through a transitional continuous mechanical pressure seal 50 to a second pressurized cooling region 106 of the pressure chamber 46. The second series of reels 122B in the second cooling region 106 of the pressure chamber 38 can also include cooling elements that help cool the packages to the appropriate cool down temperature. Upon exiting the pressure chamber 46, the continuous web 38 would be subject to any desired final processing steps as described above.

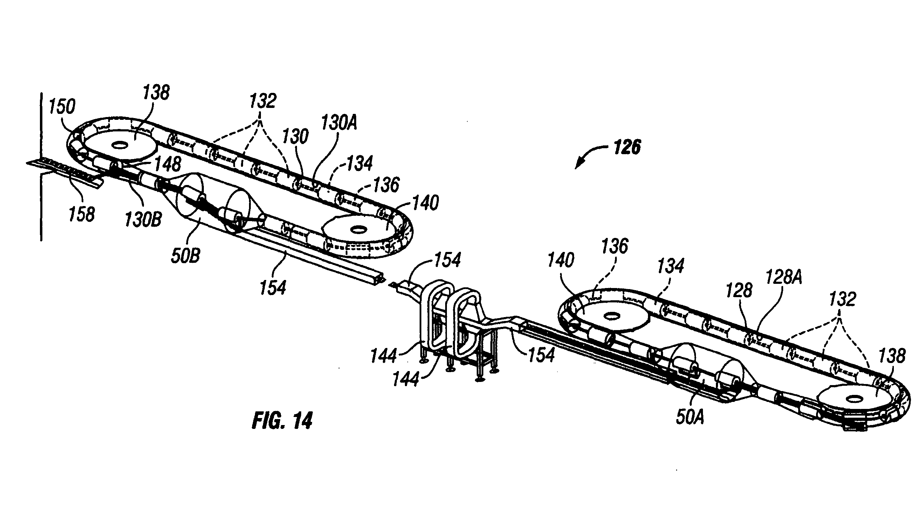

Another embodiment of the continuous mechanical pressures seal of the present invention that provides for the continuous passage of the web 38 into and out of pressure chambers is an apparatus generally described as a split baffle system 126 (Figs. 14-17). The pressure seal containment system includes at least one pressure containment loop 128; a plurality of baffle containment units 132, in which the units are connected to each other a predetermined spaced apart distance; and at least one pressure chamber 50A. The plurality of baffle units 132 are sized and shaped such that they form a air tight seal within the loop and provide for the continuous passage of a continuous web 8 of packages into and out of the at least one pressure chamber 50A.

Preferably, the split baffle pressure containment system 126 consists of two pressure containment loops of pipe 128, 130 having a plurality of sequential baffle containment units 132. Part of the pressure containment system 126 also includes a pressurized conduit that interconnects the two loops of pipe 128, 130. The continuous series of split baffles 132 move the web 38 through the first loop of pipe 128 that includes a first pressure chamber 50A for increasing the pressure surrounding the continuous web 38 of packages and after further processing, a second continuous series of split baffles 132 move the web 38 out of a second continuous loop of pipe 130 that includes a second pressure chamber 50B for returning the continuous web 38 of packages back to atmospheric pressure (Fig. 14). The split baffle system provides for continuous feeding of the web 38.

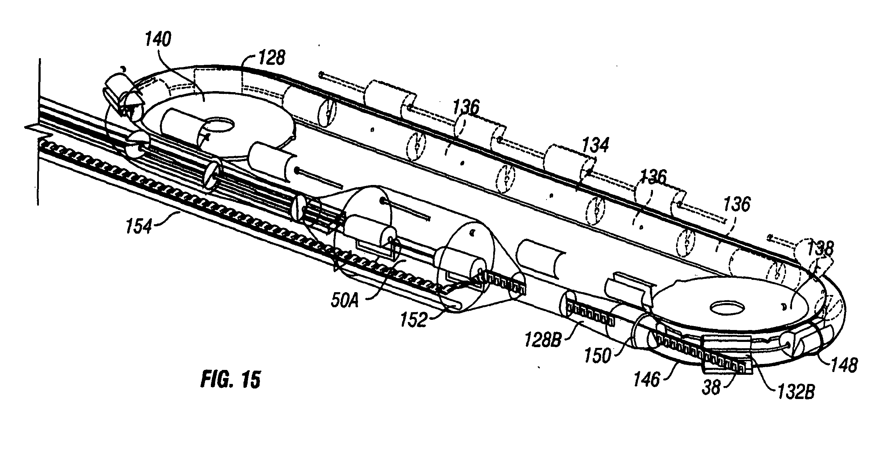

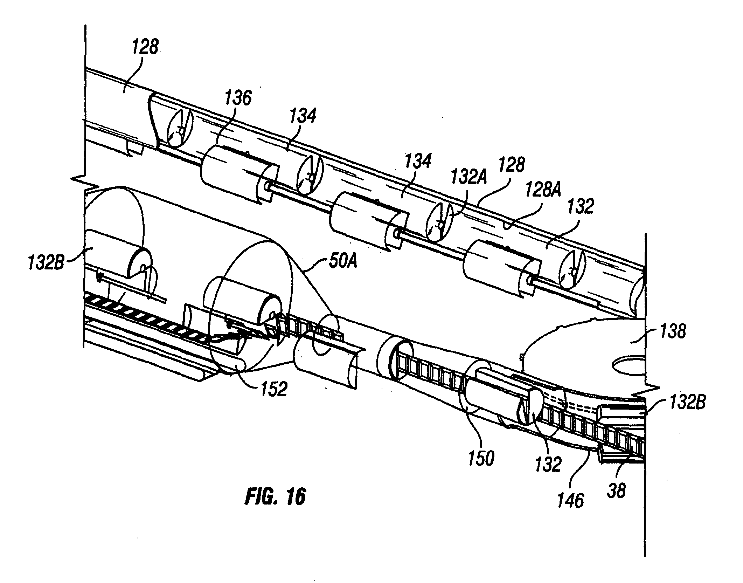

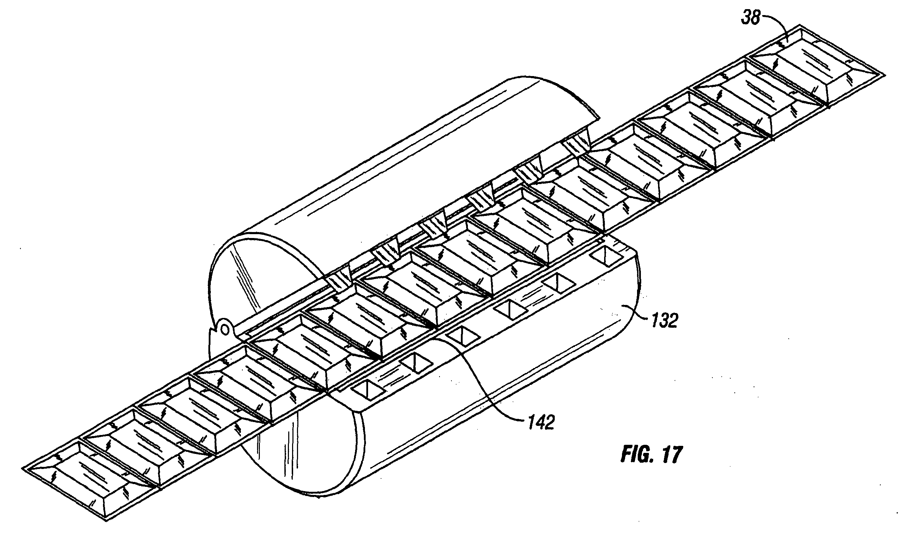

The split baffles 132 can be generally elliptical, cylindrical or rectangular in shape and preferably are configured to open like a clamshell (Figs. 16, 17). The continuous loop of baffles 134 are connected to each other by a flexible chain or rope 136 that is formed from a material that can withstand high temperatures and high pressures. In one embodiment, the continuous loop of baffles 134 can be moved through the loop of pipes 126, 130 by first and second spaced apart tracking wheels 138, 140 or other mechanisms known to one skilled in the art of moving continuous chain driven loops (Figs. 15, 16). The baffles 132 in the continuous loop 134 are spaced apart from each other a predetermined distance depending upon the size, shape and weight of the continuous web 38 of packages. The interior 142 of each baffle 132 is configured to accommodate a portion of the continuous web 38 of packages and when closed entraps the portion of the web 38 within the baffle 132 such that the continuous web 38 is moved along a portion of the pipe 128, 130 with the movement of the baffles 132 (Figs. 16, 17).

The baffle containment units 132 are preferably formed from a compressable material that can withstand high temperatures and high pressures. Examples of such material are polystyrene forms, fluorelastomer material such as VITON® by DuPont Dow Elastomers L.L.C. or fluropolymers such as TELFON® AF by DuPont. The baffles 132 also function as a pressure containment device for the system 126. The baffles 132 are sized and shaped such that the outer surfaces of the baffles 132A and the inner walls of the pipe loops 128A, 130A form an air tight seal in order to maintain the elevated pressures in the first and second pipe loops 128, 130 of the pressure containment system 126 after the continuous web 38 of packages exits the fist pressure chamber 50A and continue into the thermal cooking/sterilization apparatus 144. Alternatively, the inner walls of the pipe loops can be lined with the compressible material and the baffles would be formed from a non-compressible material.

As illustrated in Figs. 15 and 16, the first pipe loop 128 includes an open portion 146 adjacent to the first tracking wheel 138 that provides an exit 148 and an entrance 150 to the pipe loop 128. As each baffle 132 passes through the exit 148 it opens in preparation to capture a portion of the web 38. After the packages in the web 38 have been filled and sealed, and optionally processed through a pre-heating step, the continuous web 38 is directed to the open baffle 132B that captures a portion of the web 38 and thereafter closes before passing through the entrance 150 into the a first portion 128B of pipe loop 128. The closed baffles 132 pull the continuous web 38 through the first portion 128B of pipe loop 128 and into the first pressure chamber 50A. Once in the first pressure chamber 50A, each baffle 132 preferably rotates, opens and deposits the web 38 onto a conveyor 152, positioned in parallel alignment along the lower surface 154 of the pressure chamber 50A. After the baffles 132 have released the selected portions of the continuous web 38 into the pressure chamber 50A, they preferably rotate again and close as they exit the first pressure chamber 50A. As the baffles 132 continue around the first pipe loop 128, they preferable rotate so that the baffle opening is in an upward direction so that they will be in a position to open as they go through the exit 148 of the pipe 128 in order to continuously pull additional portions of the web 38 into the first pressure chamber 50A (Fig. 15).

The conveyor 152 moves the web 38 through the first pressure chamber 50A and into a pressurized conduit 154 that moves the web 38 though a thermal energy apparatus 144 for pasteurization and/or sterilization of the contents of the packages in the web 38. Preferably the thermal energy apparatus 144 utilizes microwave energy (Fig. 14).

After further processing steps, such as holding and cooling steps, the pressurized conduit 154 feeds the continuous web 38 into the second pressure chamber 50B of the second pipe loop 130 that is in a reverse configuration of the first pipe loop 128. The second pressure chamber 50B returns the packages in the web 38 to atmospheric pressure. In the second pipe loop 130, the baffles 132 open as they enter the second pressure chamber 50B and capture portions of the continuous web 38 of packages after they have returned to atmospheric pressure. The baffles 132 close as they exit the second pressure chamber 50B and pull the continuous web 38 of packages through an end portion 130B of the second pipe loop 130 and out through the exit 156 of the second pipe loop 130. Thereafter, the baffles 132 open and deposit the continuous web 38 onto another conveyor 158 that transports the web 38 to final processing. The baffles 132 close and are pulled by the first tract wheel 138A into the entrance 160 of the second pipe loop 130 to continue around the loop 130 (Fig. 14) in order to continuously move the web 38 through the second pressure chamber 50B.

In an alternate embodiment, the continuous web of packages may be overwraped by a continuous "tube" or casing of dielectric loss material, such as a formulated liquid, that contains similar dielectric loss material as the content of the products. The casing can consist of a disposable microwave transparent wrap and the continuous web of packages in which the liquid is positioned between the wrap and web. The outer wrap serves to confine the geometry of the individual packages to a specified shape (a very long stocking). The transparent wrap reduces the non-uniform heating characteristics of the surface, edge and comer portions of each package. This outer wrap would be removed before continuous package go to the cooling portion of the pressure chamber.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A method for the pasteurization and/or sterilization of products comprising the steps of:
a) providing a continuous web of individual packages containing said product;
b) moving the continuous web through an entry continuous mechanical pressure seal into a chamber having an increased pressure and temperature; and
c) moving the continuous web through an exit continuous mechanical pressure seal out of the pressure chamber.

2. The method of claim 1, wherein the entry and exit pressure seals provide for continuous feeding of the web.

3. The method of claim 1, wherein the continuous web of packages is formed from material selected from the group consisting of flexible material, semi-rigid material and combinations of flexible and semi-rigid material.

4. The method of claim 1, wherein the mechanism for moving the continuous web through the pressure seals and pressure chamber is selected from a group consisting of a clip and cable mechanism, dual carrier belts and a series of reels.

5. The method of claim 1, further including the step of accumulating portions of the continuous web prior to moving the continuous web through the first pressure seal or within the pressure chamber..

6. The method of claim 1, further including moving the continuous web through a cooling region in the chamber.

7. The method of claim 1, wherein the entry and exit pressure seals are selected from a group consisting of a rotary belt valve, a continuous rotary door pressure seal and a split baffle system.

8. The method of claim 1, further including the step of agitating the products in the continuous web while applying the increased pressure and temperature.

9. The method of claim 1, wherein the continuous web of individual flexible packages is configured to include a reinforced area for preventing the deformation or tearing of the continuous web as it is pulled through a food processing line.

10. An apparatus for the pasteurization and/or sterilization of product in a continuous web of packages comprising:
a) a chamber capable of containing an increase in pressure and temperature;
b) said chamber having a continuous mechanical pressure seal for entry and exit into and out of the chamber; and
c). a mechanism for continuous feeding of a web of packages into and out of the chamber.

11. The apparatus of claim 10, wherein one pressure seal is configured as the entry and exit pressure seals.

12. The apparatus of claim 10, wherein the chamber includes a mechanism for moving the web through the pressure seal and the chamber.

13. The apparatus of claim 12, wherein the mechanism for moving the web through the pressure seal and chamber is selected from a group consisting of a clip and cable mechanism, dual carrier belts and a series of reels.

14. The apparatus of claim 10, wherein the pressure seal is selected from a group consisting of a rotary belt valve, a continuous rotary door pressure seal and a split baffle system.

15. A pressure seal comprising:
a matched set of flexible belts;
each belt being in the shape of a continuous tube;
the belts being configured to rotate simultaneously whereby forming a rotating pressure seal.

16. A pressure seal containment system comprising:
at least one pressure containment loop;
a plurality of baffle containment units, the units being connected to each other a predetermined spaced apart distance; and
at least one pressure chamber;
wherein the plurality of baffle containment units are sized and shaped such that they form a air tight seal within the loop and provide for the continuous passage of a continuous web of packages into and out of the at least one pressure chamber.

17. The pressure seal of claim 16, wherein the containment system includes a first pressure containment loop and a first pressure chamber and a second pressure containment loop and a second pressure chamber connected to each other by a pressurized conduit.

18. The pressure seal of claim 17 wherein the first pressure chamber increases the pressure surrounding the continuous web and the second pressure chamber returns the web back to atmospheric pressure.

19. The pressure seal of claim 16, wherein the containment units are formed from a compressable material capable of withstanding high temperatures and high pressures.

20. The pressure seal of claim 16, wherein the containment units are cylindrical in shape and are configured to open like clamshell.

21. A system for multi-step food production processing comprising the steps of:
a) providing a continuous web of individual packages containing said product;
b) moving the continuous web through an entry continuous mechanical pressure seal into at least one chamber having an increased pressure and temperature; and
c) moving the continuous web through an exit continuous mechanical pressure seal out of the at least one pressure chamber.
